# EUROPÄISCHE PATENTSCHRIFT

(11) **EP 3 725 250 B1**
(45) Veröffentlichungstag und Bekanntmachung des Hinweises auf die Patenterteilung: **20.04.2022**
(21) Anmeldenummer: 19169865.3
(22) Anmeldetag: 17.04.2019
(51) Int. Cl.: A61B 18/14, A61B 18/00

(54) **ELEKTROCHIRURGISCHES INSTRUMENT MIT GELENKABDICHTUNG**
ELECTRICAL SURGICAL INSTRUMENT WITH JOINT SEALING
INSTRUMENT ÉLECTROCHIRURGICAL POURVU D'UN JOINT D'ARTICULATION ÉTANCHÉ

(43) Veröffentlichungstag der Anmeldung: 21.10.2020
(73) Patentinhaber: Erbe Elektromedizin GmbH, 72072 Tübingen (DE)
(72) Erfinder: WEILER, Rolf, 72074 Tübingen (DE)
(74) Vertreter: Rüger Abel Patentanwälte PartGmbB

(56) Entgegenhaltungen:
- EP-A1- 1 372 512
- EP-A1- 2 366 353
- WO-A1-2019/028647
- US-A- 5 484 436
- US-A1- 2014 214 019
- US-A1- 2018 132 884

## Beschreibung

Die Erfindung betrifft ein elektrochirurgisches Instrument, das beispielsweise zur Thermofusion und/oder zur Versiegelung oder Koagulation verwendet werden kann. Das elektrochirurgische Instrument hat zwei Branchen mit jeweils einem Maulteil. Jedes Maulteil weist eine Gewebekontaktfläche auf. Über die Gewebekontaktflächen kann ein Strom durch zwischen den Gewebekontaktflächen eingeklemmtes Gewebe geleitet werden, um das Gewebe beispielsweise zu versiegeln.

Solche elektrochirurgischen Instrumente sind bekannt. EP 1 372 512 B1 offenbart ein Instrument mit zwei über ein Schwenkgelenk gelenkig aneinander gelagerten Branchen, die jeweils ein Maulteil aufweisen.

US 2014/0214019 A1 beschreibt ein elektrochirurgisches Instrument mit zwei über ein Schwenkgelenk gelenkig aneinander gelagerten Branchen. Am Gelenk ist eine ringförmige Isolierscheibe zur elektrischen Isolation zwischen den Branchen angeordnet. Ein ähnliches elektrochirurgisches Instrument ist auch aus US 5,484,436 A bekannt.

Auch EP 2 366 353 A1 beschreibt ein elektrochirurgisches Instrument, dessen Branchen über ein elektrisch isoliertes Gelenk miteinander verbunden sind.

Das aus EP 1 372 512 A1 bekannte elektrochirurgische Instrument hat ein gegossenes elektrisch isolierendes Gelenk, um die Branchen voneinander zu isolieren.

Elektrochirurgische Instrumente können als Einweginstrumente oder Mehrweginstrumente bereitgestellt werden. In einigen Ländern werden auch Einweginstrumente durch spezielle Anbieter zur weiteren Verwendung aufbereitet. Mehrweginstrumente sind ohnehin für eine Mehrzahl von Anwendungen vorgesehen und müssen nach jeder Verwendung gereinigt und aufbereitet werden.

Es kann als Aufgabe der vorliegenden Erfindung angesehen werden, ein elektrochirurgisches Instrument mit zwei schwenkbar aneinander gelagerten Branchen zu schaffen, das eine vereinfachte Reinigung und/oder Sterilisation sicherstellt.

Diese Aufgabe wird durch ein elektrochirurgisches Instrument mit den Merkmalen des Patentanspruches 1 gelöst.

Das elektrochirurgische Instrument hat eine erste Branche und eine zweite Branche. Mittels mindestens eines Schwenkgelenks sind die beiden Branchen um mindestens eine Schwenkachse schwenkbar aneinander gelagert. Das mindestens eine Schwenkgelenk hat einen Lagerzapfen und eine Lageraussparung. Der Lagerzapfen ragt in die Lageraussparung hinein und definiert insbesondere die Schwenkachse. Erfindungsgemäß ist eine Dichtung zwischen den beiden Branchen an dem Schwenkgelenk vorhanden. Die Dichtung umgibt den Lagerzapfen in Umfangsrichtung um die Schwenkachse vollständig. Die Dichtung hat eine Dichtwirkung in einer Axialrichtung. Die Axialrichtung ist parallel zur Schwenkachse ausgerichtet. Die Dichtung liegt in Axialrichtung an der ersten Branche und an der zweiten Branche dichtend an. Die Anlageflächen der ersten und zweiten Branche, an denen die Dichtung anliegt, können parallel zueinander ausgerichtet sein.

Mittels der Dichtung ist das Schwenkgelenk zwischen den Branchen abgedichtet. Verunreinigungen können zwischen den Branchen nicht in einen den Lagerzapfen umgebenden Spalt eindringen bzw. das Eindringen von Verunreinigungen wird durch die Dichtung zumindest gemindert. Dennoch wird über die Dichtung sichergestellt, dass ein Spalt oder Freiraum zwischen den Branchen verbleiben kann, so dass der Bereich zwischen den Branchen für eine Reinigung ausreichend zugänglich ist.

Vorzugsweise ist die Dichtung temperaturfest bzw. temperaturbeständig für Temperaturen von mindestens 140°C. Die Dichtung ist insbesondere durchlässig für Wasserdampf und Ethylenoxid (ETO). Dadurch wird eine Sterilisation durch die Dichtung nicht behindert.

Bei einem Ausführungsbeispiel besteht die Dichtung aus Silikon.

Die Dichtung kann eine Härte von 60 Shore aufweisen.

Beispielsweise kann die Dichtung einen rechteckförmigen Querschnitt haben. Die Dichtung kann als Dichtscheibe bezeichnet werden.

Die Dichtung ist in Axialrichtung elastisch verformt, so dass die Dichtung zwischen den Branchen komprimiert ist. Dadurch entsteht eine Andrückkraft zwischen der Dichtung und den Branchen. Diese Andrückkraft sorgt für eine gute Dichtwirkung.

Die erste Branche kann zur Anlage für die Dichtung eine erste Anlagefläche aufweisen und die zweite Branche kann zur Anlage für die Dichtung eine zweite Anlagefläche aufweisen. Die Anlageflächen umschließen den Lagerzapfen jeweils an der ersten Brache bzw. der zweiten Branche.

Vorzugsweise ist wenigstens ein Abstandselement vorhanden. Das wenigstens eine Abstandselement erstreckt sich in Axialrichtung parallel zur Schwenkachse und definiert einen Axialabstand zwischen der ersten Anlagefläche der ersten Branche und der zweiten Anlagefläche der zweiten Branche. Der Axialabstand kann beispielsweise 0,1 mm bis 0,4 mm betragen. Bei einem Ausführungsbeispiel ist der Axialabstand 0,2 mm groß. Die erste und zweite Anlagefläche sind bevorzugt parallel zueinander ausgerichtet.

Der Bereich des Schwenkgelenks kann unabhängig vom Öffnungswinkel der Branchen durch die Branchen abgeschirmt bzw. verdeckt sein. Dieser Bereich kann also selbst durch beliebig weites Öffnen der Branchen nicht direkt gereinigt bzw. abgewischt werden, z.B. mit einer Bürste. Er ist also für eine mechanische Reinigung unzugänglich. Durch das wenigstens eine Abstandselement verbleibt ein definierter Spalt oder Freiraum zwischen den beiden Branchen im Bereich des Schwenkgelenks. Die Zugänglichkeit bis zur Dichtung ist daher verbessert. Außerdem kann mittels des wenigstens einen Abstandselements eine definierte axiale Kompression bzw. elastische Verformung der Dichtung zwischen den beiden Branchen vorgegeben bzw. erreicht werden. Das wenigstens eine Abstandselement kann außerdem die Gelenkstabilität verbessern und ein Verwinden und/oder Verschränken und/oder Kippen der Branchen vermeiden.

Bei einer bevorzugten Ausführungsform ist das wenigstens eine Abstandselement fest mit einer der Branchen verbunden. Das wenigstens eine Abstandselement kann integraler Bestandteil einer der beiden Branchen bzw. eines Teils einer der beiden Branchen sein. Beispielsweise kann diese eine Branche ein Kunststoffteil bzw. einen Kunststoffmantel aufweisen, der das wenigstens eine Abstandselement aufweist bzw. bildet. Alternativ kann das wenigstens eine Abstandselement auch durch ein Verbindungsverfahren mit einer der beiden Branchen verbunden werden, beispielsweise durch Schweißen, Kleben oder dergleichen.

An seinem freien Ende kann das wenigstens eine Abstandselement eine Endfläche aufweisen, die beispielsweise rechtwinklig zur Schwenkachse ausgerichtet sein kann. Die Endfläche kann sich parallel zu einer Ebene erstrecken oder zumindest abschnittsweise konvex ballig oder gekrümmt ausgebildet sein. Die Endfläche kann eine Gleitlagerfläche bilden. Mittels der Endfläche bzw. Gleitlagerfläche kann das wenigstens eine Abstandselement an der ersten Branche oder der zweiten Branche anliegen. Die Endfläche bzw. Gleitlagerfläche ist ausreichend klein, so dass eine geringe Reibung bei einer Relativbewegung der Branchen auftritt. Somit ist mittels des wenigstens einen Abstandselements ein Gleitlagerkontakt zwischen den beiden Branchen hergestellt, um die Schwenkbarkeit um die Schwenkachse zu gewährleisten. Es ist bevorzugt, wenn mindestens drei Abstandselemente und bei einem Ausführungsbeispiel genau drei Abstandselemente vorhanden sind. Die Abstandselemente können in Umfangsrichtung um die Schwenkachse mit Abstand zueinander angeordnet sein. Vorzugsweise sind die Abstandselemente in Umfangsrichtung in etwa gleichmäßig verteilt um die Schwenkachse angeordnet.

Die Dichtung hat in einem unverformten Ausgangszustand eine axiale Dicke, die zur Herstellung der elastischen Verformung größer sein kann als der Axialabstand zwischen der ersten Anlagefläche der ersten Branche und der zweiten Anlagefläche der zweiten Branche. Bei einem Ausführungsbeispiel ist die axiale Ausgangsdicke im unverformten Ausgangszustand 0,1 mm bis 0,2 mm größer als der Axialabstand. Bei einem anderen Ausführungsbeispiel ist die axiale Ausgangsdicke der Dichtung im unverformten Ausgangszustand um mindestens 25% oder um mindestens 50% größer als der Axialabstand. Die axiale Ausgangsdicke der unverformten Dichtung kann bis zu 200% größer sein als der Axialabstand.

Es ist außerdem vorteilhaft, wenn die Dichtung in wenigstens einer Vertiefung angeordnet ist. Die wenigstens eine Vertiefung kann in der ersten Branche und/oder der zweiten Branche vorhanden sein. Mittels der Vertiefung kann die Lage der Dichtung rechtwinklig bzw. radial zur Schwenkachse vorgegeben werden. Die erste Anlagefläche und/oder die zweite Anlagefläche sind durch den Boden der wenigstens einen Vertiefung gebildet. Durch die wenigstens eine Vertiefung kann der Axialabstand zwischen den Anlageflächen vergrößert werden ohne den axialen Abstand der beiden Branchen und damit die axiale Abmessung des Instruments vergrößern zu müssen. Dadurch sind größere Dicken für die Dichtung möglich, was zu einem größeren Kompressionsweg bzw. Kompressionshub zum elastischen Verformen der Dichtung in Axialrichtung führt. Dadurch kann die Dichtwirkung verbessert werden. Auf die Weise können Fertigungstoleranzen besser ausgeglichen werden. Zusätzlich ist das Instrument weniger empfindlich gegenüber verwendungsbedingten Veränderungen und äußeren Einflüssen, wie etwa Setzen und/oder Abrieb der Materialien im Gelenkbereich.

In der ersten Branche und/oder der zweiten Branche kann eine Messerführungsaussparung vorhanden sein. Die Messerführungsaussparung ist dazu eingerichtet, ein darin angeordnetes Messer in einer Bewegungsrichtung bewegbar geführt zu lagern. Die Bewegungsrichtung ist rechtwinklig zur Axialrichtung parallel zur Schwenkachse orientiert. Bei dieser Ausgestaltung kann der Lagerzapfen versetzt zur Messerführungsaussparung angeordnet sein.

Es ist vorteilhaft, wenn ein Vorspannelement vorhanden ist, das die erste Branche und die zweite Branche in Axialrichtung parallel zur Schwenkachse aufeinander zu drängt bzw. drückt. Dadurch können Toleranzen oder Verschleiß ausgeglichen werden und eine sichere und definierte Anlage bzw. Dichtwirkung erreicht werden. Dies bedeutet, dass auf die Dichtung eine Vorspannung bzw. Vorspannkraft wirkt bzw. der Gelenkbereich fixiert wird. Das Vorspannelement kann im Laufe der Zeit nachstellen, beispielsweise bei Verschleiß, Setzen des Materials, usw.

Zusätzlich zu der Dichtung zwischen den beiden Branchen sind vorzugsweise auch die Lagerspalte zwischen dem Lagerzapfen und der ersten Branche und/oder der zweiten Branche abgedichtet. Beispielsweise kann der Lagerzapfen integraler Bestandteil einer der beiden Branchen sein oder fest und dicht mit dieser Branche verbunden sein, beispielsweise verschweißt oder verklebt sein. Der Lagerzapfen kann dadurch drehfest an dieser Branche angeordnet sein. Das Eindringen von Verunreinigungen in einen Spalt um den Lagerzapfen kann durch die feste und dichte Verbindung an einem axialen Ende des Lagerzapfens verhindert werden.

Gegenüber der Branche, gegenüber der der Lagerzapfen drehbar in der Lageraussparung angeordnet ist, kann der Spalt zwischen der Lageraussparung und dem drehbar darin gelagerten Lagerzapfen durch ein zusätzliches weiteres Dichtelement abgedichtet sein. Dieses weitere Dichtelement kann analog zur Dichtung ausgebildet sein. Das weitere Dichtelement kann auch einen abdichtenden Deckel, ein Befestigungselement, insbesondere eine Befestigungsscheibe, oder dergleichen aufweisen.

Bei einer alternativen Ausführungsform kann das wenigstens eine Abstandselement am Lagerzapfen vorhanden sein oder durch einen Teil des Lagerzapfens gebildet werden.

Bei einer Ausführungsform des elektrochirurgischen Instruments kann eine Branche einen Gabelteil bilden, in den die andere Branche hineinragt, wobei der Lagerzapfen die beiden Branchen im Bereich des Gabelteils durchsetzt und schwenkbar aneinander lagert. Bei dieser Ausführungsform sind vorzugsweise zwei Dichtungen für die zwei inneren Anlageflächen der Branchen vorhanden. Die in den Gabelteil hineinragende Branche ist zwischen den Dichtungen angeordnet und damit gegenüber dem Gabelteil der anderen Branche im Bereich der Schwenkachse auf beiden Seiten abgedichtet.

Vorteilhafte Ausgestaltungen der Erfindung ergeben sich aus den abhängigen Patentansprüchen, der Beschreibung und den Zeichnungen. Nachfolgend werden bevorzugte Ausführungsbeispiele der Erfindung anhand der beigefügten Zeichnungen im Einzelnen erläutert. Es zeigen:

Figur 1 eine schematische Seitenansicht eines Ausführungsbeispiels eines elektrochirurgischen Instruments mit zwei schwenkbar aneinander gelagerten Branchen,

Figur 2 eine schematische Seitenansicht eines weiteren Ausführungsbeispiels eines elektrochirurgischen Instruments mit zwei schwenkbar aneinander gelagerten Branchen,

Figur 3 eine schematische Explosionsdarstellung des elektrochirurgischen Instruments aus Figur 2,

Figur 4 eine perspektivische Schnittdarstellung durch ein Ausführungsbeispiels eine Schwenkgelenks zur schwenkbaren Lagerung der Branchen aus den Figuren 1 bis 3,

Figur 5 eine schematische perspektivische Schnittdarstellung eines weiteren Ausführungsbeispiels eines Schwenkgelenks zur schwenkbaren Lagerung der Branchen aus den Figuren 1 bis 3,

Figur 6 eine schematische Ansicht einer Branche im Bereich des Schwenkgelenks mit Blick auf die der jeweils anderen Branche zugewandten Fläche aufweisend einen Messerführungskanal und Abstandshalteelemente,

Figuren 7 bis 11 jeweils schematische Schnittdarstellungen entlang der Schwenkachse unterschiedlicher Ausführungsbeispiele von Schwenkgelenken zur schwenkbaren Lagerung der Branchen aus den Figuren 1 bis 3,

Figur 12 eine Prinzipdarstellung zur Veranschaulichung der axialen Kompression einer zwischen den Branchen angeordneten Dichtung.

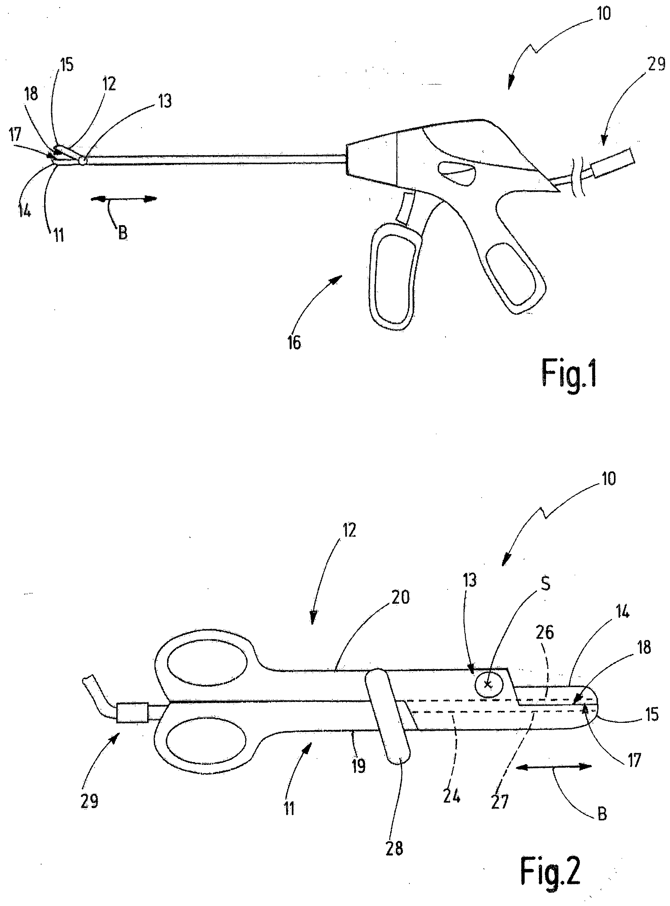

In den Figuren 1 und 2 sind stark schematisiert unterschiedliche Ausführungsbeispiele eines elektrochirurgischen Instruments 10 veranschaulicht, das beispielsgemäß als biopolares Instrument ausgebildet ist. Das elektrochirurgische Instrument 10 hat eine erste Branche 11 und eine zweite Branche 12, die mittels eines Schwenkgelenks 13 um eine Schwenkachse S schwenkbar aneinander gelagert sind. Die Richtung parallel zur Schwenkachse S wird als Axialrichtung A bezeichnet.

Bei dem in Figur 1 dargestellten Ausführungsbeispiel sind von den Branchen 11, 12 im Wesentlichen die sich ausgehend von der Schwenkachse S bzw. dem Schwenkgelenk 13 in einem distalen Bereich erstreckenden Maulteile 14, 15 veranschaulicht. Das erste Maulteil 14 und/oder das zweite Maulteil 15 können schwenkbar gelagert sein. Zum Öffnen bzw. Schließen der Maulteile 14, 15 weist das Instrument 10 aus Figur 1 eine Handhabungseinheit 16 auf. Mittels der Handhabungseinheit 16 können auch weitere Aktionen ausgeführt werden, beispielsweise das Anlegen einer Spannung zwischen einer ersten Gewebekontaktfläche 17 am ersten Maulteil 14 und einer zweiten Gewebekontaktfläche 18 am zweiten Maulteil 15. Dabei kann ein Stromfluss durch ein Gewebe verursacht werden, das zwischen den beiden Gewebekontaktflächen 17, 18 eingeklemmt bzw. gehalten wird.

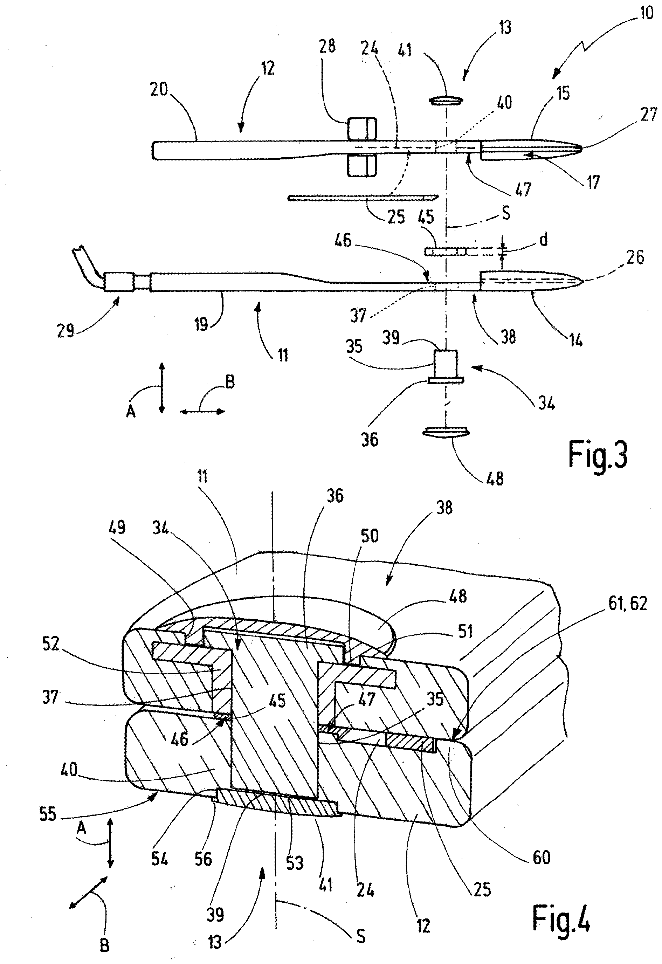

In Figur 2 ist ein weiteres Ausführungsbeispiel eines elektrochirurgischen Instruments 10 veranschaulicht, das scherenartig ausgebildet ist. Figur 3 zeigt das Ausführungsbeispiel aus Figur 2 in einer schematischen Explosionsdarstellung. Die erste Branche 11 weist bei diesem Ausführungsbeispiel ausgehend von der Schwenkachse S in Richtung zum distalen Ende das erste Maulteil 14 und ausgehend von der Schwenkachse S in Richtung zum proximalen Ende ein erstes Handhabungsteil 19 auf. Entsprechend hat die zweite Branche 12 ausgehend von der Schwenkachse S in Richtung zum distalen Ende das zweite Maulteil 15 und ausgehend von der Schwenkachse S in Richtung zum proximalen Ende ein zweites Handhabungsteil 20.

In einer der Branchen und beispielsgemäß in der ersten Branche 11 ist außerdem eine Messerführungsaussparung 24 vorhanden, die sich im Bereich des Schwenkgelenks 13 und ausgehend vom Schwenkgelenk 13 weg in Richtung zum proximalen Ende des ersten Handhabungsteils 19 hin erstreckt. In der Messerführungsaussparung 24 ist ein Messer 25 (Figur 3) in einer Bewegungsrichtung B geführt bewegbar gelagert. Die Messerführungsaussparung 24 setzt sich in einer ersten Führungsvertiefung 26 im ersten Maulteil 14 und in einer zweiten Führungsvertiefung 27 im zweiten Maulteil 15 fort. Das Messer 25 kann durch eine Messerbetätigung 28 in Bewegungsrichtung B aus der Messerführungsaussparung 24 entlang der Führungsvertiefungen 26, 27 bewegt werden. Zwischen den Gewebekontaktflächen 17, 18 gehaltenes bzw. eingeklemmtes Gewebe kann dann durch das Messer 25 durchtrennt werden.

Wie es in Figur 2 zu erkennen ist, ist das Schwenkgelenk 13 bzw. die Schwenkachse S quer zur Bewegungsrichtung B versetzt zur Messerführungsaussparung 24 angeordnet. Die Messerführungsaussparung 24 erstreckt sich somit in Bewegungsrichtung B am Schwenkgelenk 13 vorbei.

In den Figuren 1 und 2 ist außerdem eine elektrische Verbindungseinrichtung 29 veranschaulicht, die dazu eingerichtet ist, mit einem externen Stecker oder einem anderen geeigneten Anschluss verbunden zu werden. Die Verbindungseinrichtung 29 ist bei scherenartigen elektrochirurgischen Instrumenten vorzugsweise an einer der Branchen und beispielsgemäß an der ersten Branche 11 am proximalen Ende und somit am ersten Handhabungsteil 19 angeordnet. Über nicht näher dargestellte Leiter innerhalb der ersten Branche 11 werden die beiden Gewebekontaktflächen 17, 18 elektrisch mit der elektrischen Verbindungseinrichtung 29 verbunden.

Der Stromkreis kann bei einem Ausführungsbeispiel über eine weitere am Körper des Patienten befindliche Neutralelektrode geschlossen werden (monopolares HF-Klemmeninstrument). Dabei weisen beiden Gewebekontaktflächen 17,18 dasselbe elektrische Potential auf. Der Gelenkbereich muss intern nicht elektrisch isoliert sein.

Bei einem Instrument 10, das als bipolares Instrument ausgebildet ist, wird der Stromkreis zwischen den beiden Gewebekontaktflächen 17,18 über das daran anliegende Gewebe geschlossen. Über die Verbindungseinrichtung 29 werden also zwei Leiter bis zum Gelenk vorgeführt. Die Strom- und/oder Potentialübertragung bis zur Gewebekontaktfläche 18 der zweiten Branche 12 wird im Gelenkbereich realisiert. Dabei ist vorzugsweise im Gelenkbereich eine elektrische Isolation vorhanden, insbesondere um die Potentialtrennung sicherzustellen.

Beim Ausführungsbeispiel nach Figur 1 ist die elektrische Verbindungseinrichtung 29 an der Handhabungseinheit 16 angeordnet und die elektrischen Leiter erstrecken sich ausgehend von der Handhabungseinheit 16 bis zu den Gewebekontaktflächen 17, 18. Der Aufbau eines Ausführungsbeispiels des Schwenkgelenks 13 ist in der Explosionsdarstellung in Figur 3 und in einem perspektivischen Schnittbild entlang der Schwenkachse S und rechtwinklig zur Bewegungsrichtung B in Figur 4 veranschaulicht. Das Schwenkgelenk 13 weist bei diesem Ausführungsbeispiel einen separaten Lagerzapfen 34 auf. Der Lagerzapfen 34 hat einen sich koaxial zur Schwenkachse S erstreckenden kreiszylindrischen Achsabschnitt 35. An einem axialen Ende hat der Lagerzapfen 34 einen Kopf 36, der eine größere Abmessung rechtwinklig zur Schwenkachse S aufweist als der Achsabschnitt 35. Der Kopf 36 kann beispielsweise durch ein scheibenförmiges und insbesondere kreisringscheibenförmiges Teil des Lagerzapfens 34 gebildet sein. Der Achsabschnitt 35 und der Kopf 36 können integral ausgebildet sein oder durch separate Bauteile gebildet sein, die fest miteinander verbunden sind.

Die erste Branche 11 hat eine Lageraussparung 37, durch die der Achsabschnitt 35 des Lagerzapfens 34 drehbar gelagert hindurchragt. Der Kopf 36 liegt an einer von der zweiten Branche 12 abgewandten Außenfläche 38 an der ersten Branche 11 an. Das dem Kopf 36 entgegengesetzte freie Ende 39 des Lagerzapfens 34 ragt in ein Durchgangsloch 40 der zweiten Branche 12 hinein oder durch das Durchgangsloch 40 hindurch. Das freie Ende 39 ist mit einem Abschlussteil 41 verbunden, das quer zur Schwenkachse S zumindest teilweise eine größere Abmessung aufweist als das Durchgangsloch 40. Das Abschlussteil 41 und/oder der Lagerzapfen 34 kann beispielsweise durch Schweißen und/oder Kleben und/oder einen Presssitz und/oder eine andere geeignete Verbindung fest mit der zweiten Branche 12 verbunden werden. Dadurch wird der Lagerzapfen 34 gegen eine ungewollte Bewegung in Axialrichtung A parallel zur Schwenkachse S gesichert. Das Abschlussteil 41 kann beispielsweise durch Schweißen und/oder Kleben und/oder einen Presssitz und/oder eine andere geeignete Verbindung fest mit dem Lagerzapfen 34 verbunden werden. Bei dem in den Figuren 3 und 4 veranschaulichten Ausführungsbeispiel hat das Abschlussteil 41 eine scheibenförmige oder plattenförmige oder kappenförmige Gestalt.

Die zweite Branche 12 und der Lagerzapfen 34 sind elektrisch miteinander auf demselben Potential. Beispielsgemäß bewirkt das Abschlussteil 41 eine elektrische Isolation des freien Endes 39 des Lagerzapfens 34 nach außen und dient als Berührschutz. Die elektrische Isolation des freien Endes 39 des Lagerzapfens 34 kann auch durch andere Maßnahmen erreicht werden, z.B. durch das Abdecken des freien Endes 39 mit Isolationsmaterial, wie etwa einem Klebstoff. Der Klebstoff kann nach seiner Aushärtung eine Form gleich oder ähnlich dem Abschlussteil 41 annehmen, beispielsweise eine kappenförmige Gestalt. Bei einer solchen Ausführungsform muss das Abschlussteil 41 keine Sicherungswirkung des Lagerzapfens 34 gegen eine ungewollte Bewegung in Axialrichtung A parallel zur Schwenkachse S aufweisen. Der Achsabschnitt 35 des Lagerzapfens 34 kann direkt am Durchgangsloch 40 der zweiten Branche 12 fixiert bzw. gesichert sein.

Das Abschlussteil 41 ist optional. Es kann auch nur eine rein elektrisch isolierende Wirkung besitzen. In diesem Fall ist die Gelenkachse 12 direkt mit der Branche 12 verbunden, z.B. verschweißt. Bei dieser Ausführungsform entspricht das Abschlusselement 41 im Wesentlichen einer Kappe. Alternativ oder zusätzlich kann das Abschlussteil 41 auch die vorstehend geschilderten mechanischen Aufgaben erfüllen und das Gelenk axial zusammenhalten.

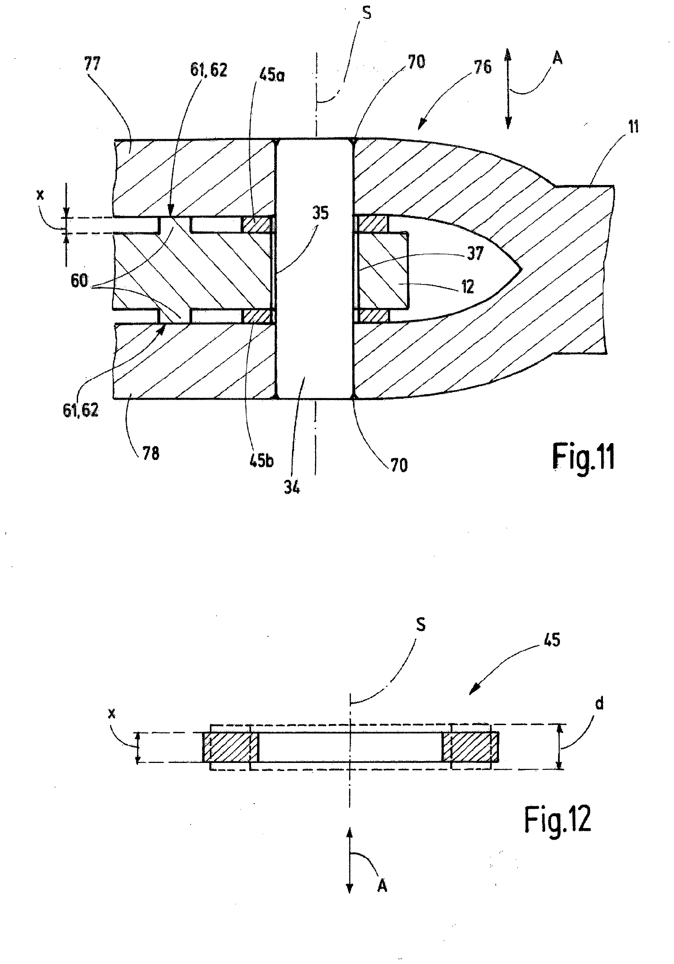

Das elektrochirurgische Instrument 10 weist außerdem eine Dichtung 45 auf, die in einer Umfangsrichtung um die Schwenkachse S ringförmig geschlossen ist. Die Dichtung 45 kann eine kreisringförmige Kontur aufweisen. In Figur 12 ist die Dichtung 45 schematisch im Querschnitt veranschaulicht. Gestrichelt dargestellt ist die Form der Dichtung 45 in einem elastisch unverformten Ausgangszustand. In Axialrichtung A parallel zur Schwenkachse hat die Dichtung 45 dabei eine axiale Dicke d. Im Einbauzustand zwischen den beiden Branchen 11, 12 wird die Dichtung 45 in Axialrichtung A komprimiert und deren Dicke verringert. Durch dieses Komprimieren in Axialrichtung A nimmt die Querschnittsabmessung der Dichtung 45 radial zur Schwenkachse S zu, wie es in Figur 12 schematisch veranschaulicht ist. Die mit durchgezogenen Linien und den schraffierten Querschnittsflächen dargestellte Dichtung 45 entspricht dem in Axialrichtung A komprimierten Zustand.

Die Dichtung 45 liegt in Axialrichtung an einer ersten Anlagefläche 46 an der ersten Branche 11 und auf der in Axialrichtung A entgegengesetzten Seite an einer zweiten Anlagefläche 47 der zweiten Branche 12 dichtend an. Die erste Anlagefläche 46 umgibt die Lageraussparung 37 ringförmig. Die zweite Anlagefläche 47 umgibt das Durchgangsloch 40 ringförmig. Die erste Anlagefläche 46 und die zweite Anlagefläche 47 sind einander zugewandt und in Axialrichtung A mit einem Axialabstand x zueinander angeordnet. Somit weist die Dichtung 45 in Axialrichtung A in komprimiertem Zustand eine Dicke auf, die dem Axialabstand x zwischen der ersten Anlagefläche 46 und der zweiten Anlagefläche 47 entspricht.

Der Axialabstand x beträgt vorzugsweise zumindest 0,1 mm und höchstens 0,4 mm. Bei dem hier dargestellten Ausführungsbeispiel beträgt der Axialabstand etwa 0,2 mm. Im unverformten Ausgangszustand hat die Dichtung 45 eine axiale Dicke d, die um beispielsweise 0,1 mm bis 0,2 mm größer ist als der Axialabstand x (Figur 12). Vorzugsweise ist die axiale Dicke d im unverformten Ausgangszustand mindestens 25% größer oder mindestens 50% größer oder mindestens 100% größer als der Axialabstand x. Es ist weiter bevorzugt, wenn die axiale Dicke d dem unverformten Ausgangszustand um maximal 200% größer ist als der Axialabstand x.

Die Dichtung 45 hat beispielsgemäß einen rechteckförmigen Querschnitt und kann daher auch als Dichtscheibe bezeichnet werden. Ihre Härte beträgt bei einem Ausführungsbeispiel 60 Shore. Vorzugsweise besteht die Dichtung 45 aus einem Kunststoffmaterial oder einem Verbundwerkstoff oder aus Silikon. Die Dichtung 45 ist durchlässig für Wasserdampf und Ethylenoxid (ETO). Das Material der Dichtung 45 ist temperaturbeständig bzw. temperaturfest für Temperaturen im Bereich von 140°C bis zu 250°C oder 300°C oder 400°C.

Mittels der Dichtung 45 wird der Lagerbereich zwischen den beiden Branchen abgedichtet. Verunreinigungen können nicht oder nur in geringerer Menge zwischen den beiden Branchen 11, 12 in den Lagerspalt zwischen dem Lagerzapfen 34 und der Lageraussparung 37 bzw. dem Durchgangsloch 40 gelangen.

Um das Eindringen von Verunreinigungen auch an den axialen Enden, also dem Kopf 36 und dem freien Ende 39 zu verhindern, wird der Lagerzapfen 34 beim Ausführungsbeispiel im Bereich des axialen Endes 39 abgedichtet, beispielsweise durch das kappenförmige Abschlussteil 41 und/oder eine Klebeverbindung und/oder eine Schweißverbindung mit der zweiten Branche 12. Das Abschlussteil 41 kann dichtend eng an der zweiten Branche 12 anliegen und ist vorzugsweise durch eine Klebeverbindung, Haftverbindung oder Schweißverbindung dicht mit der zweiten Branche 12 verbunden. Beispielsweise kann eine in Umfangsrichtung ringsumlaufende Klebeverbindung oder Schweißverbindung vorhanden sein.

Um den Lagerzapfen 34 im Bereich des Kopfes 36 abzudichten, ist bei diesem Ausführungsbeispiel eine Kappe 48 vorhanden, die den Kopf 36 abdeckt und dichtend an der ersten Branche 11 und beispielsgemäß der Außenfläche 38 der ersten Branche 11 anliegt. Die Kappe 48 kann beispielsweise durch eine Klebeverbindung, Haftverbindung, Schweißverbindung oder dergleichen fest mit der ersten Branche 11 verbunden sein, um dadurch das Eindringen von Verunreinigungen zwischen der Kappe 48 und der ersten Branche 11 zu verhindern. Beispielsweise kann eine in Umfangsrichtung ringsumlaufende Klebeverbindung oder Schweißverbindung vorhanden sein. Der Lagerzapfen 34 kann relativ zur Kappe 48 um die Schwenkachse S drehbar angeordnet sein.

In der Außenfläche 38 ist eine erste Aussparung 49 angebracht, in der der Kopf 36 und ein den Kopf 36 in Umfangsrichtung um die Schwenkachse S umschließender Ring 50 der Kappe 48 zumindest teilweise aufgenommen ist. Die Kappe 48 hat einen Umfangsrand 51, der ausgehend vom Ring 50 radial nach außen von der Schwenkachse S wegragt und außerhalb der ersten Aussparung 49 an der Außenfläche 38 der ersten Branche 11 anliegt.

Zur Drehlagerung kann zwischen dem Achsabschnitt 35 und der ersten Branche 11 eine Lagerbuchse 52 vorhanden sein. Diese Lagerbuchse 52 ist beispielsgemäß gegenüber der ersten Branche 11 elektrisch isoliert. Einer der von der Verbindungseinrichtung 29 ausgehenden elektrischen Leiter ist mit der Lagerbuchse 52 verbunden. Die Lagerbuchse 52 bildet gegenüber dem Lagerzapfen 34 einen Schleifkontakt. Über den Lagerzapfen 34 kann somit eine elektrische Verbindung zur zweiten Branche 12 hergestellt werden.

Das Abschlussteil 41 hat einen zentralen Abschnitt 53, der beispielsgemäß eine zylindrische Form hat und in einer zweiten Aussparung 54 in einer von der ersten Branche 11 abgewandten Außenfläche 55 der zweiten Branche 12 aufgenommen ist. Ein Umfangsrand 56 des Abschlussteils 41 umschließt die Schwenkachse S und ragt ausgehend vom zentralen Abschnitt 53 radial nach außen von der Schwenkachs S weg. Der Umfangsrand 56 ist außerhalb der zweiten Aussparung 54 angeordnet und liegt an der Außenfläche 55 der zweiten Branche 12 an.

Die Branchen 11 und 12 weißen vorzugsweise einen Metallkern auf, der für die Struktursteifigkeit sorgt und auch als Stromleiter verwendet werden kann. Die Außenflächen der Branchen 11 und 12 also auch die Außenflächen 38 und 55 sind beispielsgemäß elektrisch nicht leitend und gegenüber dem Metallkern elektrisch isoliert. Der Metallkern ist beispielsweise beschichtet, umgossen oder umspritzt.

Somit ist das Schwenkgelenk 13 sowohl von den axialen Enden her, als auch zwischen den Branchen 11, 12 - mittels der Dichtung 45 - gegen das Eindringen von Verschmutzungen in den Gelenkbereich gesichert. Das Reinigen eines derartigen elektrochirurgischen Instruments 10 vereinfacht sich dadurch.

Um einen definierten Axialabstand x zwischen der ersten Anlagefläche 46 und der zweiten Anlagefläche 47 zu gewährleisten, weist die erste Branche 11 und/oder die zweite Branche 12 wenigstens ein Abstandselement 60 auf, das sich in Axialrichtung A erstreckt. Bei dem in Figur 4 gezeigten Ausführungsbeispiel ist eines der vorhandenen Abstandselemente 60 in der Querschnittsfläche zu erkennen, das integraler Bestandteil der zweiten Branche 12 ist und sich von der zweiten Branche 12 weg zur ersten Branche 11 hin erstreckt.

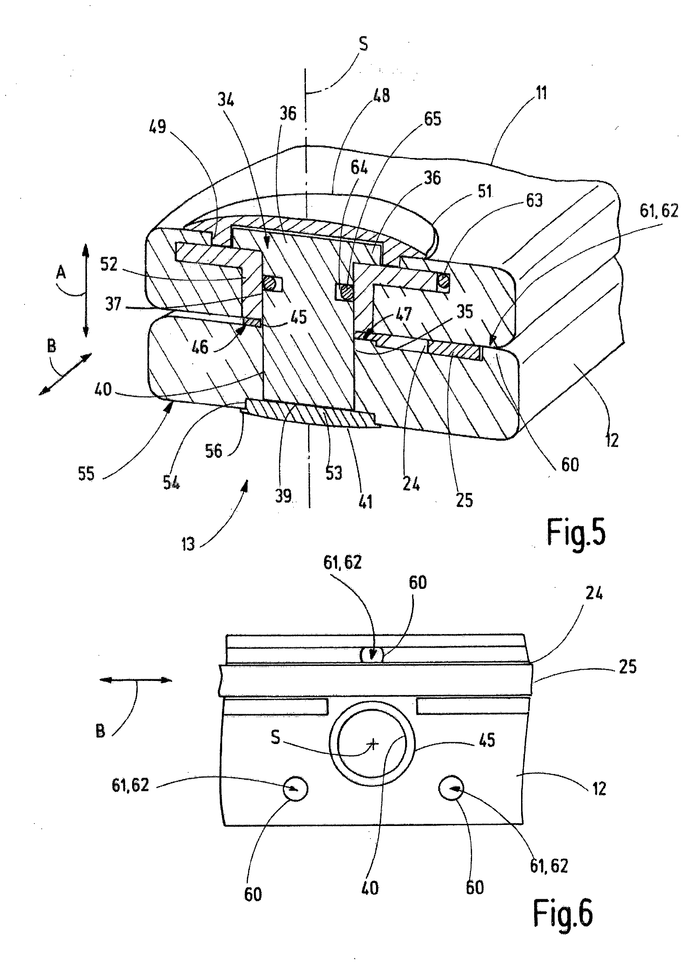

Jedes Abstandselement 60 ist fest mit einer der beiden Branchen 11 oder 12 verbunden. Bei dem hier veranschaulichten Ausführungsbeispiel sind alle Abstandselemente 60 fest mit der zweiten Branche 12 verbunden und vorzugsweise integraler Bestandteil der zweiten Branche 12, beispielsweise eines Kunststoffkörpers bzw. einer Kunststoffummantelung der zweiten Branche 12. Jedes Abstandselement 60 kann auch Teil eines Metallkerns einer der Branchen 11, 12 sein. Der Metallkern ist dann beschichtet, umgossen, umspritzt oder auf andere Weise nach außen elektrisch isoliert, beispielsweise durch einen Kunststoffkörper oder eine Kunststoffummantelung. Vorzugsweise sind mehrere Abstandselemente 60 vorhanden. Wie es in Figur 6 zu erkennen ist, sind beispielsgemäß drei Abstandselemente 60 an der zweiten Branche 12 vorhanden, die in Umfangsrichtung um die Schwenkachse S mit Abstand zueinander angeordnet sind. Der Radialabstand zwischen der Schwenkachse S und den Abstandselementen 60 kann jeweils gleich groß oder auch verschieden groß sein. Bevorzugt sind die Abstandselemente 60 in Umfangsrichtung um die Schwenkachse S entlang einer gemeinsamen Umkreislinie angeordnet und gleichmäßig um die Schwenkachse S verteilt angeordnet. Auf diese Weise sind die Branchen 11, 12 bzw. die Maulteile 14, 15 der beiden Branchen 11, 12 gegen ein unbeabsichtigtes Kippen relativ zueinander gesichert. Durch die Abstände in Umfangsrichtung zwischen den Abstandselementen 60 und durch einen vorzugsweise vorhandenen Radialabstand zur Dichtung 45 besteht ausreichend Raum zum Durchspülen mit einer Spülflüssigkeit, wodurch die Reinigung verbessert wird.

Bei einem Ausführungsbeispiel können zumindest einige der Abstandselemente 60 eine zylindrische Kontur aufweisen.

Jedes Abstandselement 60 hat eine Endfläche 61, die beim Ausführungsbeispiel der ersten Branche 11 zugewandt ist und an der die erste Branche 11 gleitend anliegt. Die Endfläche 61 erstreckt sich beispielsgemäß in einer Ebene rechtwinklig zur Schwenkachse S. Die Endfläche kann alternativ dazu auch konvex gekrümmt sein. Die Endfläche 61 jedes Abstandselementes 60 bildet eine Gleitlagerfläche 62 für die erste Branche 11. Beim Schwenken der beiden Branchen 11, 12 um die Schwenkachse S relativ zueinander gleitet die erste Branche 11 an den Gleitlagerflächen 62 der Abstandselemente 60 jeweils auf einer kreisbogenförmigen Bahn um die Schwenkachse S.

Es versteht sich, dass die Kontur, Anzahl und Anordnung der Abstandselemente 60 bei anderen Ausführungsbeispielen auch anders sein kann, als es in Figur 6 veranschaulicht ist.

In Figur 6 ist außerdem zu erkennen, dass die Messerführungsaussparung 24 zwischen einem der Abstandselemente 60 und der Schwenkachse S in Bewegungsrichtung B verläuft. Diese Anordnung ist vorteilhaft, weil der Abstand der Messerführungsaussparung 24 von der Schwenkachse S so klein wie möglich gewählt werden soll. Der verfügbare Raum zwischen dem Außenumfang der Dichtung 45 und der Messerführungsaussparung 24 bzw. dem Messer 25 kann somit nicht ausreichend sein für das Anordnen eines Abstandselements 60, das daher beispielsgemäß auf der anderen Seite der Messerführungsaussparung 24 vorhanden ist.

In Figur 5 ist ein gegenüber der in Figur 4 gezeigten Ausführung abgewandeltes Ausführungsbeispiel veranschaulicht. Insoweit die Ausführungsbeispiele übereinstimmen, wird auf die vorstehende Beschreibung verwiesen. Zusätzlich zu dem in Figur 4 gezeigten und bisher beschriebenen Ausführungsbeispiel ist eine Möglichkeit der elektrischen Kontaktierung eines Leiters 63 mit dem Lagerzapfen 34 veranschaulicht. Der Leiter 63 erstreckt sich beispielsgemäß entlang der ersten Branche 11 bis zur Lagerbuchse 52 und ist elektrisch leitend mit der Lagerbuchse 52 verbunden. Die Lagerbuchse 52 ist elektrisch leitfähig. Wegen des Schwenklagerspiels zwischen der Lagerbuchse 52 und dem Lagerzapfen 34 kann bevorzugt in einer Ringaussparung 64 ein Federkontakt 65 angeordnet sein, der einerseits elektrisch leitend mit dem Lagerzapfen 34 verbunden ist und andererseits radial nach außen von der Schwenkachse S gegen eine Innenfläche der Lagerbuchse 52 vorgespannt ist.

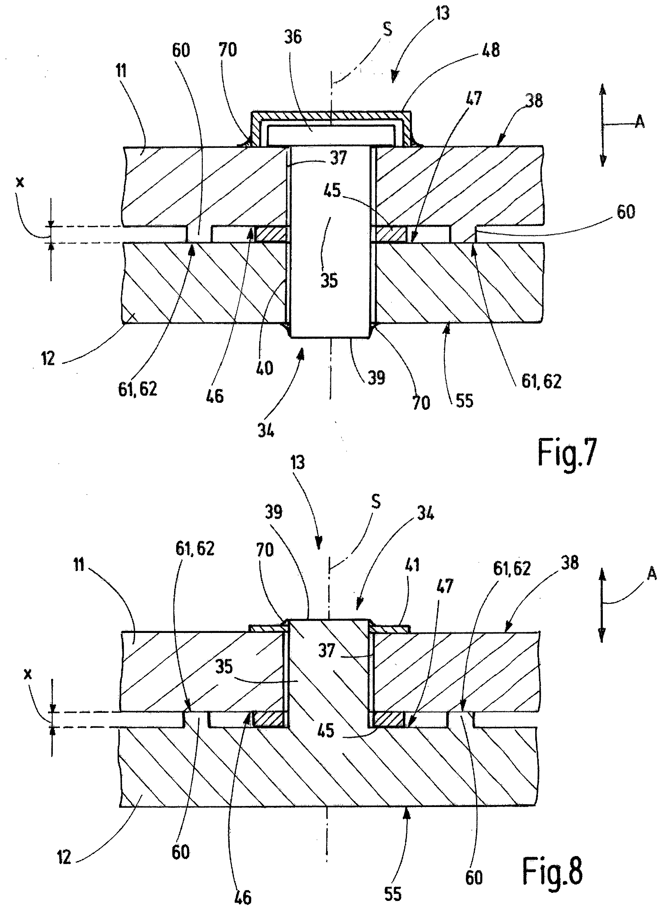

In den Figuren 7 bis 11 sind weitere Ausführungsmöglichkeiten zur Realisierung des Schwenklagers 13 stark schematisiert in einem Querschnitt entlang der Schwenkachse S rechtwinklig zur Bewegungsrichtung B veranschaulicht.

Bei dem in Figur 7 gezeigten Ausführungsbeispiel ist das freie Ende 39 des Lagerzapfens 34 durch eine ringsumlaufende Verbindung 70, beispielsweise eine Schweißnaht oder eine Klebeverbindung mit der zweiten Branche 12 verbunden, so dass das Eindringen von Verschmutzungen in den Spalt zwischen dem Lagerzapfen 34 und dem Durchgangsloch 40 vermieden ist. Eine weitere ringsumlaufende Verbindung 70 ist zwischen der ersten Branche 11 und der Kappe 48 vorhanden, die ebenfalls als Schweißnaht oder Klebeverbindung ausgeführt sein kann.

Die Abstandselemente 60 sind bei diesem Ausführungsbeispiel im Unterschied zu den bisher beschriebenen Ausführungsformen an der ersten Branche 60 vorhanden bzw. integraler Bestandteil davon.

Die in Figur 8 schematisch veranschaulichte Ausführungsform sieht vor, dass das wenigstens eine Abstandselement 60 in der zweiten Branche 12 angeordnet oder integraler Bestandteil davon ist. Der Lagerzapfen 34 ist bei diesem Ausführungsbespiel ebenfalls integraler Bestandteil einer der Branchen 11, 12 und beispielsgemäß der zweiten Branche 12. Das Durchgangsloch 40 und das Kopfteil 36 entfallen. Der Achsabschnitt 35 ragt beispielgemäß von der zweiten Anlagefläche 47 weg durch die Lageraussparung 37 zum freien Ende 39 hin. Das freie Ende 39 ragt bei diesem Ausführungsbeispiel aus der Lageraussparung 37 heraus und ist durch ein beispielsgemäß scheibenförmiges Abschlussteil 41 und/oder den Achsabschnitt 35 gesichert. Das scheibenförmige Abschlussteil 41 ist über eine ringsumlaufende Verbindung 70 mit dem freien Ende 39 bzw. dem Achsabschnitt 35 des Lagerzapfens 34 dicht und fest verbunden. Das Sicherungselement 41 liegt über eine Ringfläche mit radial ausreichender Abmessung an der Außenseite 38 der ersten Branche 11 unter Bildung einer Gleitverbindung dichtend an. Dadurch ist das Eindringen von Verschmutzungen in den Lagerspalt zwischen dem Achsabschnitt 35 und der Lageraussparung 37 zumindest gehemmt oder verhindert.

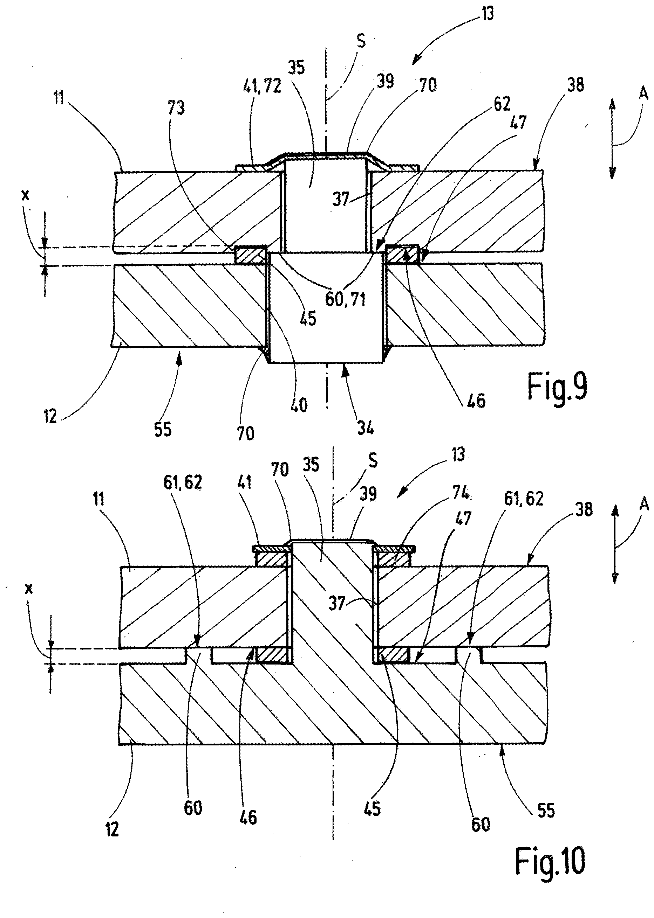

Das in Figur 9 schematisch veranschaulichte Ausführungsbeispiel des Schwenklagers 13 weist einen Lagerzapfen 34 auf, an dem das wenigstens eine Abstandselement 60 in Form von wenigstens einem Radialvorsprung 71 gebildet ist, der gegenüber dem Achsabschnitt 35 radial von der Schwenkachse S nach außen vorspringt. Bei dem in Figur 9 veranschaulichten Ausführungsbeispiel ist ein einziger ringsumlaufend ausgebildeter Radialvorsprung 71 vorhanden, der somit eine Ringschulter bildet. Es könnten auch mehrere in Umfangsrichtung um die Schwenkachse S mit Abstand zueinander angeordnete Radialvorsprünge 71 vorhanden sein, insbesondere wenigstens drei solcher Radialvorsprünge 71.

Bei wenigstens einem weiteren Ausführungsbeispiel befindet sich das wenigstens eine Abstandselement 60 radial zur Schwenkachse S gesehen zwischen der Dichtung 45 und der Schwenkachse S. Das wenigstens eine Abstandselement 60 kann von der Dichtung 45 umschlossen sein und sich sozusagen im abgedichteten Bereich des Schwenkgelenks 13 befinden. Diese Ausgestaltung ist auch durch eine Abwandlung der Ausführungsformen aus der Figuren 7, 8 und 10 möglich, in dem das wenigstens eine Abstandselement 60 radial weiter innen angeordnet wird als die Dichtung 45 bzw. zentral angeordnet wird.

An dem wenigstens einen Radialvorsprung 71 ist die Gleitfläche 62 vorhanden, die zur gleitenden Anlage mit einer der Branchen beispielsgemäß mit der ersten Branche 11 dient. Im Unterschied zu den bisher beschriebenen Ausführungsbeispielen ist somit das wenigstens eine Abstandselement 60 nicht an einer der Branchen, sondern am Lagerzapfen 34 vorhanden. Das Abstandselement 60 befindet sich im durch die Dichtung 45 abgedichteten Bereich. Die Dichtung 45 ist radial zur Schwenkachse S gesehen weiter von der Schwenkachse S entfernt als der Radialvorsprung 71.

Im Bereich des freien Endes 39 ragt der Achsabschnitt 35 des Lagerzapfens 34 aus der Lageraussparung 37 heraus und ist dort ähnlich wie beim Ausführungsbeispiel nach Figur 8 durch eine ringsumlaufende Verbindung 70 mit einem Abschlussteil 41 dichtend verbunden. Im Unterschied zum Ausführungsbeispiel nach Figur 8 ist das Abschlussteil 41 bei diesem Ausführungsbeispiel als Vorspannelement 72 ausgebildet, das eine Vorspannkraft zwischen den beiden Branchen 11, 12 erzeugt. Das Vorspannelement 72 erzeugt eine Vorspannkraft in Axialrichtung A auf den Lagerzapfen 34 und drängt die Gleitlagerfläche 62 dadurch gegen die erste Branche 11. An seinem dem freien Ende 39 entgegengesetzten Ende ist der Lagerzapfen 34 durch eine ringsumlaufende Verbindung 70 fest mit der zweiten Branche 12 verbunden, insbesondere im Bereich der Außenfläche 55 der zweiten Branche. Mittels des Vorspannelements 72 kann somit sichergestellt werden, das auch aufgrund von Herstellungstoleranzen und/oder Verschleiß und/oder Setzprozessen der Axialabstand x entweder aufrecht erhalten bleibt oder bewusst verringert wird um die Dichtwirkung der Dichtung 45 weiterhin sicherzustellen.

In Figur 9 ist außerdem zu erkennen, dass die Dichtung 45 in einer Vertiefung 73 angeordnet sein kann. Die Vertiefung 73 ist beim Ausführungsbeispiel in der ersten Branche 11 vorhanden. Die Vertiefung 73 ist zur zweiten Anlagefläche 47 der zweiten Branche 12 hin offen. Der Boden der Vertiefung 73 bildet zumindest einen Abschnitt der ersten Anlagefläche 46. Zusätzlich oder alternativ könnte auch in der zweiten Branche 12 eine Vertiefung 73 vorhanden sein. Die wenigstens eine Vertiefung 73 hat den Vorteil, dass die axiale Dicke d der Dichtung 45 in unverformtem Ausgangszustand größer gewählt werden kann und dadurch ein größerer Kompressionsweg bzw. Kompressionshub zur Verfügung steht, ohne die axialen Abmessungen des elektrochirurgischen Instruments in Axialrichtung A vergrößern zu müssen. Außerdem kann durch die wenigstens eine Vertiefung 73 die Position der Dichtung 45 rechtwinklig zur Schwenkachse S bzw. zum Lagerzapfen 34 definiert und aufrechterhalten werden.

Bei der Ausführungsform nach Fig. 9 können zusätzlich zu dem am Lagerzapfen 34 ausgebildeten Abstandselement 60 noch weitere Maßnahmen zur Stabilisierung des Schwenkgelenks 13 z.B. gegen ein unerwünschtes Kippen vorhanden sein. Beispielsweise können zusätzliche Abstandselemente 60 vorhanden sein, wie sie in den Figuren 4 bis 8 dargestellt sind. Solche zusätzlichen Abstandselemente 60 hätten dann beispielsweise lediglich die Aufgabe der Stabilisierung, aber nicht mehr der Begrenzung bzw. Einstellung der axialen Kompression der Dichtung 45.

Das in Figur 10 veranschaulichte Ausführungsbeispiel des Schwenklagers 13 entspricht im Wesentlichen dem Ausführungsbeispiel gemäß Figur 8. Im Unterschied zum Ausführungsbeispiel nach Figur 8 ist bei der in Figur 10 gezeigten Ausführungsform ein weiteres Dichtelement 74 vorhanden. Das weitere Dichtelement 74 ist beispielsgemäß zwischen dem Abschlussteil 41 und der ersten Branche 11 bzw. der Außenfläche 38 der ersten Branche 11 angeordnet. Das weitere Dichtelement 74 kann entsprechend der Dichtung 45 ausgebildet sein. Das weitere Dichtelement 74 umschließt das aus der Lageraussparung 37 herausragende freie Ende 39 bzw. den Achsabschnitt 35 des Lagerzapfens 34 vollständig und dichtet den Spalt zwischen dem Abschlussteil 41 und der ersten Branche 11 ab. Im Übrigen kann auf die Beschreibung zu Figur 8 verwiesen werden. Das weitere Dichtelement 74 ist auch als weitere, abgewandelte Ausführungsform des in Figur 9 gezeigten Ausführungsbeispiels verwendbar

Die bisher beschriebenen Ausführungsformen des Schwenklagers 13 treffen eine einseitige Lagerung. Die erste Branche 11 und die zweite Branche 12 sind dabei entlang der Schwenkachse S nebeneinander angeordnet ohne ineinander zu greifen. Bei dem in Figur 11 dargestellten Ausführungsbeispiel hat die eine Branche und beispielsgemäß die erste Branche 11 im Bereich des Schwenklagers 13 einen Gabelteil 76 mit einem ersten Arm 77 und einem zweiten Arm 78, die in Axialrichtung A mit Abstand zueinander angeordnet sind. Der Lagerzapfen 34 durchsetzt die beiden Arme 77, 78 und ist am jeweiligen axialen Ende durch eine ringsumlaufende Verbindung 70 - insbesondere Schweißverbindung oder Klebeverbindung - mit dem Arm 77 bzw. 78 fest und dicht verbunden. In dem Freiraum zwischen den Armen 77, 78 ragt die zweite Branche 12 mit der Lageraussparung 37 hinein, die von dem Achsabschnitt 35 des Lagerzapfens 34 durchsetzt wird. Eine erste Dichtung 45a ist zwischen der zweiten Branche 12 und dem ersten Arm 77 und eine zweite Dichtung 45b ist zwischen der zweiten Branche 12 und dem zweiten Arm 78 angeordnet und liegt jeweils dichtend zwischen dem betreffenden Arm 77 bzw. 78 einerseits und der zweiten Branche 12 andererseits an. Zumindest zwischen einem der beiden Arme 77, 78 und beispielsgemäß zwischen beiden Armen 77, 78 und der zweiten Branche 12 kann optional wenigstens ein Abstandselement 60 angeordnet sein, um den Axialabstand x analog zu den bisher beschriebenen Ausführungsbeispielen vorzugeben und die axiale Kompression der Dichtungen 45a, 45b zu definieren.

Es versteht sich, dass die in den Zeichnungen dargestellten Ausführungsbeispiele der Ausgestaltung des Schwenklagers 13 auch miteinander kombiniert werden können. Beispielsweise kann bei jedem Ausführungsbeispiel wenigstens eine Vertiefung 73 zur Aufnahme der Dichtung 45 vorhanden sein.

Bei jedem Ausführungsbeispiel des Schwenklagers 13 kann ein Vorspannelement 72 vorhanden sein, um die beiden Branchen 11, 12 in Axialrichtung A mit einer Vorspannkraft aufeinander zu vorzuspannen bzw. gegeneinander zu drängen. Außerdem kann auch bei jedem Ausführungsbeispiel ein weiteres Dichtelement 74 vorhanden sein, um den Bereich eines axialen Endes und insbesondere des freien Endes 39 des Lagerzapfens 34 gegenüber dem Lagerspalt zwischen dem Achsabschnitt 35 und der Lageraussparung 37 abzudichten.

Die Erfindung betrifft ein elektrochirurgisches Instrument 10 mit zwei Branchen 11, 12, die mittels eines Schwenkgelenks 13 schwenkbar aneinander gelagert sind. Das Schwenkgelenk 13 hat einen Lagerzapfen 34 und eine Lageraussparung 37. Der Lagerzapfen 34 ist insbesondere drehfest mit einer der Branchen 11 oder 12 verbunden und drehbar in der Lageraussparung 37 der jeweils anderen Branche 12 bzw. 11 gelagert. Zwischen den Branchen 11, 12 ist eine Dichtung 45 vorhanden, die dichtend an beiden Branchen 11, 12 anliegt und den Lagerzapfen 34 in Umfangsrichtung vollständig umschließt. Vorzugsweise ist der Lagerzapfen 34 an seinen beiden axialen Enden dichtend mit der jeweiligen Branche 11, 12 verbunden. Dadurch wird das Eindringen von Verschmutzungen in den Bereich zwischen dem Lagerzapfen 34 und der Lageraussparung 37 insbesondere von allen Seiten bzw. Zugangsmöglichkeiten verhindert oder zumindest reduziert.

### Bezugszeichenliste:

- 10: elektrochirurgisches Instrument
- 11: erste Branche
- 12: zweite Branche
- 13: Schwenkgelenk
- 14: erstes Maulteil
- 15: zweites Maulteil
- 16: Handhabungseinheit
- 17: erste Gewebekontaktfläche
- 18: zweite Gewebekontaktfläche
- 19: erstes Handhabungsteil
- 20: zweites Handhabungsteil

- 24: Messerführungsaussparung
- 25: Messer
- 26: erste Führungsvertiefung
- 27: zweite Führungsvertiefung
- 28: Messerbetätigung
- 29: elektrische Verbindungseinrichtung

- 34: Lagerzapfen
- 35: Achsabschnitt des Lagerzapfens
- 36: Kopf des Lagerzapfens
- 37: Lageraussparung
- 38: Außenfläche der ersten Branche
- 39: freies Ende des Lagerzapfens
- 40: Durchgangsloch
- 41: Abschlussteil

- 45: Dichtung
- 46: erste Anlagefläche
- 47: zweite Anlagefläche
- 48: Kappe
- 49: erste Aussparung
- 50: Ring der Kappe
- 51: Umfangsrand
- 52: Lagerbuchse
- 53: zentraler Abschnitt
- 54: zweite Aussparung
- 55: Außenfläche der zweiten Branche

- 60: Abstandselement
- 61: Endfläche
- 62: Gleitlagerfläche
- 63: Leiter
- 64: Ringaussparung
- 65: Federkontakt

- 70: Verbindung
- 71: Radialvorsprung
- 72: Vorspannelement
- 73: Vertiefung
- 74: weiteres Dichtelement

- 76: Gabelteil
- 77: erster Arm
- 78: zweiter Arm

- A: Axialrichtung
- d: axiale Dicke
- S: Schwenkachse
- x: Axialabstand

## Patentansprüche

1. Elektrochirurgisches Instrument (10) mit einer ersten Branche (11) und einer zweiten Branche (12), die mittels eines Schwenkgelenks (13) um eine Schwenkachse (S) schwenkbar aneinander gelagert sind, wobei das Schwenkgelenk (13) einen Lagerzapfen (34) und eine Lageraussparung (37) aufweist,
**dadurch gekennzeichnet, dass** das elektrochirurgische Instrument (10) außerdem eine Dichtung (45) aufweist, die den Lagerzapfen (34) in Umfangsrichtung um die Schwenkachse (S) vollständig umschließt und an der ersten Branche (11) und der zweiten Branche (12) dichtend anliegt, wobei die Dichtung (45) zwischen den Branchen (11, 12) in einer Axialrichtung (A) parallel zur Schwenkachse (S) komprimiert und elastisch verformt ist.

2. Elektrochirurgisches Instrument nach Anspruch 1,
**dadurch gekennzeichnet, dass** die erste Branche (11) eine erste Anlagefläche (46) für die Dichtung (45) aufweist und dass die zweite Branche (12) eine zweite Anlagefläche (47) für die Dichtung (45) aufweist.

3. Elektrochirurgisches Instrument nach Anspruch 2,
**dadurch gekennzeichnet, dass** wenigstens ein Abstandselement (60) vorhanden ist, das sich in einer Axialrichtung (A) parallel zur Schwenkachse (S) erstreckt und einen Axialabstand (x) zwischen der ersten Anlagefläche (46) und der zweiten Anlagefläche (47) definiert.

4. Elektrochirurgisches Instrument nach Anspruch 3,
**dadurch gekennzeichnet, dass** das wenigstens eine Abstandselement (60) fest mit einer der Branchen (11, 12) verbunden ist.

5. Elektrochirurgisches Instrument nach Anspruch 3 oder 4, **dadurch gekennzeichnet, dass** das wenigstens eine Abstandselement (60) an einem freien Ende eine Endfläche (61) aufweist, die eine Gleitlagerfläche (62) bildet und an der ersten Branche (11) oder der zweiten Branche (12) anliegt.

6. Elektrochirurgisches Instrument nach einem der Ansprüche 3 bis 5,
**dadurch gekennzeichnet, dass** mindestens drei Abstandselemente (60) vorhanden sind, die in Umfangsrichtung um die Schwenkachse (S) mit Abstand zueinander angeordnet sind.

7. Elektrochirurgisches Instrument nach einem der Ansprüche 3 bis 6,
**dadurch gekennzeichnet, dass** die Dichtung (45) in einem unverformten Ausgangszustand eine axiale Dicke (d) aufweist, die größer ist als der Axialabstand (x).

8. Elektrochirurgisches Instrument nach einem der vorhergehenden Ansprüche,
**dadurch gekennzeichnet, dass** in der ersten Branche (11) und/oder der zweiten Branche (12) eine Vertiefung (73) zur Aufnahme der Dichtung (45) vorhanden ist.

9. Elektrochirurgisches Instrument nach einem der vorhergehenden Ansprüche,
**dadurch gekennzeichnet, dass** die Dichtung (45) einen rechteckförmigen Querschnitt aufweist.

10. Elektrochirurgisches Instrument nach einem der vorhergehenden Ansprüche,
**dadurch gekennzeichnet, dass** in der ersten Branche (11) und/oder der zweiten Branche (12) eine Messerführungsaussparung (24) vorhandenen ist, in der ein Messer (25) in einer Bewegungsrichtung (B) bewegbar geführt gelagert ist.

11. Elektrochirurgisches Instrument nach Anspruch 10,
**dadurch gekennzeichnet, dass** der Lagerzapfen (34) versetzt zur Messerführungsaussparung (24) angeordnet ist.

12. Elektrochirurgisches Instrument nach einem der vorhergehenden Ansprüche,
**dadurch gekennzeichnet, dass** ein Vorspannelement (72) vorhanden ist, das die erste Branche (11) und die zweite Branche (12) in einer Axialrichtung (A) parallel zur Schwenkachse (S) aufeinander zu drängt.

13. Elektrochirurgisches Instrument nach einem der vorhergehenden Ansprüche,
**dadurch gekennzeichnet, dass** der Lagerzapfen (34) drehfest mit der ersten Branche (11) oder der zweiten Branche (12) verbunden ist und drehbar in einer Lageraussparung (37) der jeweils anderen Branche (12 bzw. 11) angeordnet ist.

14. Elektrochirurgisches Instrument nach Anspruch 13,
**dadurch gekennzeichnet, dass** der Bereich zwischen der Lageraussparung (37) und dem Lagerzapfen (34) mittels wenigstens einem weiteren Dichtelement (74) abgedichtet ist.

## Claims

1. Electrosurgical instrument (10) with a first jaw (11) and a second jaw (12) that are pivotably arranged about a pivot axis (S) at each other by a pivot joint (13), wherein the pivot joint (13) comprises a support pin (34) and a support cavity (37),
**characterized in that** the electrosurgical instrument (10) further comprises a seal (45) that completely surrounds the support pin (34) in circumferential direction around the pivot axis (S) and sealingly abuts against the first jaw (11) and the second jaw (12), whereby the seal (45) is compacted and elastically deformed in an axial direction (A) parallel to the pivot axis (S) between the jaws (11, 12).

2. Electrosurgical instrument according to claim 1, **characterized in that** the first jaw (11) comprises a first abutment surface (46) for the seal (45) and wherein the second jaw (12) comprises a second abutment surface (47) for the seal (45).

3. Electrosurgical instrument according to claim 2, **characterized in that** at least one distance element (60) is provided that extends in an axial direction (A) parallel to the pivot axis (S) and defines an axial distance (X) between the first abutment surface (46) and the second abutment surface (47).

4. Electrosurgical instrument according to claim 3, **characterized in that** the at least one distance element (60) is rigidly connected with one of the jaws (11, 12) .

5. Electrosurgical instrument according to claim 3 or 4, **characterized in that** the at least one distance element comprises an end surface (61) at a free end that forms a friction bearing surface (62) and abuts against the first jaw (11) or the second jaw (12).

6. Electrosurgical instrument according to any of the claims 3 to 5, **characterized in that** at least three distance elements (60) are provided that are arranged with distance to each other in circumferential direction around the pivot axis (S).

7. Electrosurgical instrument according to any of the claims 3 to 6, **characterized in that** the seal (45) comprises an axial thickness (d) in the undeformed initial condition that is larger than the axial distance (X).

8. Electrosurgical instrument according to any of the preceding claims, **characterized in that** a depression (73) for location of the seal (45) is provided in the first jaw (11) and/or the second jaw (12).

9. Electrosurgical instrument according to any of the preceding claims, **characterized in that** the seal (45) has a rectangular cross-section.

10. Electrosurgical instrument according to any of the preceding claims, **characterized in that** a knife guide cavity (24) is provided in the first jaw (11) and/or the second jaw (12), in which a knife (25) is movably supported in a movement direction (B) in a guided manner.

11. Electrosurgical instrument according to claim 10, **characterized in that** the support pin (34) is arranged offset to the knife guide cavity (24).

12. Electrosurgical instrument according to any of the preceding claims, **characterized in that** a biasing element (72) is provided that urges the first jaw (11) and the second jaw (12) in an axial direction (A) parallel to the pivot axis (S) toward each other.

13. Electrosurgical instrument according to any of the preceding claims, **characterized in that** the support pin (34) is torque-proof connected with the first jaw (11) or the second jaw (12) and is rotatably supported in a support cavity (37) of the respective other jaw (12 or 11) .

14. Electrosurgical instrument according to claim 13, **characterized in that** the region between the support cavity (37) and the support pin (34) is sealed by means of at least one additional seal element (74).

## Revendications

1. Instrument électrochirurgical (10) comprenant une première branche (11) et une deuxième branche (12) qui sont montées l'une sur l'autre à l'aide d'une articulation pivotante (13), avec possibilité de pivotement autour d'un axe de pivotement (S), l'articulation pivotante (13) présentant un tourillon (34) et un évidement de palier (37),
**caractérisé en ce que** l'instrument électrochirurgical (10) présente en outre un joint d'étanchéité (45) qui entoure le tourillon (34) complètement dans le sens périphérique, autour de l'axe de pivotement (S), et est appliqué de manière étanche contre la première branche (11) et la deuxième branche (12), le joint (45) étant comprimé et déformé élastiquement entre les branches (11, 12), dans une direction axiale parallèle à l'axe de pivotement (S).

2. Instrument électrochirurgical selon la revendication 1, **caractérisé en ce que** la première branche (11) présente une première surface d'appui (46) pour le joint d'étanchéité (45), et **en ce que** la deuxième branche (12) présente une deuxième surface d'appui (47) pour le joint (45).

3. Instrument électrochirurgical selon la revendication 2, **caractérisé en ce qu'**il est prévu au moins un élément d'espacement (60) qui s'étend dans une direction axiale (A), parallèlement à l'axe de pivotement (S), et définit une distance axiale (x) entre la première surface d'appui (46) et la deuxième surface d'appui (47).

4. Instrument électrochirurgical selon la revendication 3, **caractérisé en ce que** l'élément d'espacement (60), au nombre d'au moins un, est relié solidement à l'une des branches (11, 12).

5. Instrument électrochirurgical selon la revendication 3 ou 4, **caractérisé en ce que** l'élément d'espacement (60), au nombre d'au moins un, présente à une extrémité libre une face d'extrémité (61) qui constitue une surface d'appui lisse (62) et est appliquée contre la première branche (11) ou la deuxième branche (12).

6. Instrument électrochirurgical selon l'une des revendications 3 à 5, **caractérisé en ce qu'**il est prévu au moins trois éléments d'espacement (60) qui sont disposés à distance les uns des autres dans la direction périphérique autour de l'axe de pivotement (S).

7. Instrument électrochirurgical selon l'une des revendications 3 à 6, **caractérisé en ce que** dans un état initial non déformé, le joint d'étanchéité (45) présente une épaisseur axiale (d) qui est supérieure à la distance axiale (x).

8. Instrument électrochirurgical selon l'une des revendications précédentes, **caractérisé en ce qu'**une partie en retrait (73), destinée à accueillir le joint d'étanchéité (45), est prévue dans la première branche (11) et/ou la deuxième branche (12).

9. Instrument électrochirurgical selon l'une des revendications précédentes, **caractérisé en ce que** le joint d'étanchéité (45) présente une section rectangulaire.

10. Instrument électrochirurgical selon l'une des revendications précédentes, **caractérisé en ce qu'**un évidement de guidage de lame (24) est prévu dans la première branche (11) et/ou la deuxième branche (12), dans lequel une lame (25) est guidée de façon mobile dans une direction de déplacement (B).

11. Instrument électrochirurgical selon la revendication 10, **caractérisé en ce que** le tourillon (34) est disposé avec un décalage par rapport à l'évidement de guidage de lame (24).

12. Instrument électrochirurgical selon l'une des revendications précédentes, **caractérisé en ce qu'**il est prévu un élément de précontrainte (72) qui pousse la première branche (11) et la deuxième branche (12) l'une en direction de l'autre, dans une direction axiale (A) qui est parallèle à l'axe de pivotement (S).

13. Instrument électrochirurgical selon l'une des revendications précédentes, **caractérisé en ce que** le tourillon (34) est lié de façon solidaire en rotation à la première branche (11) ou la deuxième branche (12) et est disposé avec possibilité de rotation dans un évidement de palier (37) respectivement de l'autre branche (12 ou 11).

14. Instrument électrochirurgical selon la revendication 13, **caractérisé en ce que** la région entre l'évidement de palier (37) et le tourillon (34) est rendue étanche à l'aide d'au moins un élément d'étanchéité (74) supplémentaire.
